(19) Europäisches Patentamt
European Patent Office
Office européen des brevets

(11) EP 0 565 241 B1

(12) EUROPEAN PATENT SPECIFICATION

(45) Date of publication and mention of the grant of the patent:
**24.04.1996 Bulletin 1996/17**

(51) Int Cl.[6]: **C07H 13/04**, A61K 31/70, C12N 9/64

(21) Application number: **93301878.0**

(22) Date of filing: **12.03.1993**

(54) **Oligosaccharides**

Oligosaccharides

Oligosaccharide

(84) Designated Contracting States:
**AT BE CH DE DK ES FR GB GR IE IT LI LU NL PT SE**

(30) Priority: **12.03.1992 US 849868**

(43) Date of publication of application:
**13.10.1993 Bulletin 1993/41**

(73) Proprietor: **ELI LILLY AND COMPANY**
**Indianapolis, Indiana 46285 (US)**

(72) Inventor: **Yan, Sau-Chi Betty**
**Indianapolis, Indiana 46240 (US)**

(74) Representative: **Hudson, Christopher Mark et al**
**Lilly Industries Limited**
**European Patent Operations**
**Erl Wood Manor**
**Windlesham Surrey GU20 6PH (GB)**

(56) References cited:
**US-A- 5 009 889**

- **BIO/TECHNOLOGY vol. 8, July 1990, pages 655 - 661 S.C.B. YAN ET AL. 'Characterization and Novel Purification of Recombinant Human Protein C fromThree Mammalian Cell Lines'**
- **ARCH. BIOCHEM. BIOPHYS. vol. 290, no. 2, 1 November 1991, pages 474 - 483 M. OHTA ET AL. 'Further Characterization of Allergenically Active Oligosaccharitols Isolated from a Sea Squirt H-Antigen'**
- **CELL vol. 63, 30 November 1990, pages 861 - 863 B.K. BRANDLEY ET AL. 'Carbohydrate Ligands of the LEC Cell Adhesion Molecules'**
- **FEBS LETTERS vol. 314, no. 3, December 1992, pages 389 - 394 A.A. BERGWERFF ET AL. 'Human urokinase contains GalNAc(1-4)[Fuc(1-3)]GlcNAc(1-2) as a novel terminal element in N-linked carbohydrate chains'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention relates to molecular biology, particularly to methods for the production of novel carbohydrate structures in human kidney 293 cells. These novel carbohydrate structures can be used to treat inflammation and can also be used to reduce cell adhesion.

Human Kidney 293 cells are adenovirus-transformed cells useful in producing recombinant proteins. The cells have been found to be particularly useful for producing blood protein products such as human protein C, human protein S and thrombomodulin, as well as tissue plasminogen activator, various derivative forms of human protein C and renin. Human protein C produced in 293 cells demonstrates a glycosylation pattern which is markedly distinct from the glycosylation pattern found on plasma-derived human protein C. This invention relates to the fact that the human protein C molecules produced in 293 cells have now been shown to contain novel N-linked oligosaccharides which contain non-reducing terminal branches of GalNAcβ(1-4)(Fucα(1-3))GlcNAc. These structures are capable of binding with high affinity to certain selectins, which are cell adhesion receptors of the human immune system. This specific ligand affinity demonstrates that the novel oligosaccharides produced by 293 cells are useful in the reduction of cell adhesion and tissue inflammation.

US-A-5009889 discloses the use of protein C (not from 293 kidney cells) in the treatment of inflammation and its anticoagulant activity is discussed. Arch. Biophys. 1991, vol. 290, p. 474-483, also discloses that the GalNAcβ1-4 (Fucα1-3) GlcNAcβ1-2 non reducing terminal saccharide sequence is involved in allergic response, which may involve oligosaccharide adhesion.

For purposes of the present invention, as disclosed and claimed herein, the following terms are as defined below.

Asn - asparagine
Gal - galactose.
Fuc - fucose.
GalNAc - N-acetylgalactosamine.
GlcNAc - N-acetylglucosamine.
Man - Mannose.
NeuAc - N-acetylneuraminic acid.

Figure 1. shows a chromatograph of the major carbohydrates found on the human protein C molecules produced in 293 cells. The major peaks for which carbohydrate structures have been elucidated are marked with numbers.

Peptides containing oligosaccharides with the structure GalNacβ(1-4)(Fucα(1-3))GlcNAc can be produced by incubating Human Kidney 293 cells expressing a peptide capable of being glycosylated under conditions suitable for expression of the peptide, then recovering the peptide from the cell or cell culture supernatent. Human Protein C molecules produced in 293 cells contain these structures on the non-reducing terminal branches of N-linked oligosaccharides. The invention also comprises methods for further isolating the novel oligosaccharides from the peptide produced in 293 cells. The peptides and/or oligosaccharides so produced can be used to act as high affinity ligands of certain cell adhesion receptors. Such ligand specificity demonstrates that the peptides and novel oligosaccharides may be used to prevent or reduce cell adhesion and thereby prevent or reduce tissue inflammation.

Human Kidney 293 cells (ATCC CRL 1573) are transformed primary embryonal human kidney cells which contain and express the transforming gene of Adenovirus 5. These cells have been used to express several important gene products and have been used by a number of different laboratories both in academia and industry. For example, Yan, U.S. Patent No. 4,981,952 and Bang et. al., U.S. Patent No. 4,992,373, both disclose the use of the 293 cell line to produce human protein C. It has now been discovered that the 293 cell line contains the enzymatic machinery to create novel and useful oligosaccharides on peptides produced in the cell line. These novel oligosaccharides, as well as methods for making and using them, comprise the present invention.

Human Protein C (HPC) circulates in the plasma as a zymogen of a serine protease. Protein C is activated by thrombin in complex with the endothelial cell surface receptor, thrombomodulin. The activated form of human protein C (aPC) has potent anticoagulant activity due to its ability to inactivate factors Va and VIIIa. For purposes of the present disclosure, the term "human protein C" refers to both the zymogen and activated forms of the molecule. Protein C plays a critical role in the regulation of thrombin generation and may be effective in the treatment of a number of thrombotic diseases. Human Protein C is post-translationally modified by N-glycosylation at four sites on the molecule; specifically at position Asn 97 on the light chain, and at positions Asn 248, Asn 313, and Asn 329 on the heavy chain. While the full carbohydrate structures of plasma-derived HPC have not been reported, the oligosaccharides found on HPC recombinantly-produced in 293 cells (rHPC) are quite distinct from those found on plasma-derived HPC. For example, rHPC has a five-fold higher fucose content and a two-fold lower NeuAc content in comparison to plasma-derived HPC. In addition, there are 2.6 moles of GalNAc per mole of rHPC while plasma-derived HPC contains no GalNAc.

Oligosaccharides may not only be linked to a peptide at an Asn residue. Oligosaccharides may be linked to peptides

via N-glycosylation or O-glycosylation. Oligosaccharides are also found within glycolipids and proteoglycans and may be added to membrane bound proteins via glypiation. For the purposes of this disclosure, the term "capable of being glycosylated" is not limited to N-glycosylation of an Asn residue, but rather includes all mechanisms by which peptides or lipids are glycosylated in vivo.

The major oligosaccharides found on rHPC produced in 293 cells display the profile shown in Figure 1 upon fingerprinting on the Dionex HPAE-PAD system. The oligosaccharide corresponding to major peak 1 is a neutral, biantennary oligosaccharide with both antennae containing the structure of GalNAcβ(1-4)(Fucα(1-3))GlcNAcα(1-2)-linked to the fucosylated chitobiose-trimannosyl core. Several of the other ten oligosaccharides studied and disclosed herein also display the novel N-linked structure containing GalNAc. The predicted structures of the oligosaccharides of peaks 2, 7 and 11 also contain the novel structure on at least one branch of the fucosylated chitobiose-trimannosyl core. In addition, the structures of the oligosaccharides of peaks 15, 20 and 23 contain GalNAc residues in 1-4 linkage. These novel oligosaccharide structures are produced by the 293 cell line because the cells contain the specific enzymes necessary for production of these substrates.

Human Kidney 293 cells appear to have both α(2-3) and α(2-6) sialyltransferases. The α(2-6) sialyltransferase is specific for sialylating GalNAc residues while the α(2-3) sialyltransferase is specific for sialylating Gal residues. The α (1-3) fucosyltransferase expressed by 293 cells can act on a substrate containing GalNAc. The action of the α(2-6) sialyltransferase and the α(1-3) fucosyltransferase appear to be mutually exclusive on any individual branch of the oligosaccharide, therefore once the GalNAc is sialylated the α(1-3) fucosylation of the GalNAc residue in the same antenna is precluded. Conversely, once the GalNAc is fucosylated, the same GalNAc residue on the same antenna cannot be sialylated by the α(2-6) sialyltransferase.

The 293 cells produce recombinant glycoproteins with less heterogeneity in the carbohydrate portion of the molecule because 293 cells express very high levels of α (1-6) fucosyltransferase activity. The linkage analysis of all 25 oligosaccharide peaks isolated from rHPC revealed only 4,6-linked GlcNAc with a reducing end. No 4-linked GlcNAc with a reducing end was detected. From this it is apparent that the chitobiose cores of all the N-linked oligosaccharides were virtually 100% fucosylated. The elucidation of the 11 major oligosaccharides on 293 cell-derived rHPC accounts for the unusual glycosyl content of the rHPC as reported by Yan et al., 1990, BioTechnology 8:655-660. The high fucose content of the molecule can be accounted for by the fact that the chitobiose core of all of the N-linked oligosaccharides are fucosylated and also by the fact that the GlcNAc residues in the antenna(e) of four of the 11 major N-linked oligosaccharides are fucosylated. With the exception of the oligosaccharide found in peak 2, the GalNAc residues in rHPC were all found in N-glycosylated oligosaccharides and were located only in the antennae position where normally only Gal is found in N-linked complex type oligosaccharides. For purposes of this disclosure, the terms antenna and branch can include the plural antennae and branches and vice versa.

The action of the fucosyltransferase found in 293 cells and the resulting α(1-3)fucosylation of the GlcNAc residues of oligosaccharides made in the cell line demonstrate yet another facet of the present invention. Structures containing α(1-3)fucosylated GlcNAc are useful ligands for the binding of selectins. Selectins are a type of adhesion receptor of the immune system. The adhesion of leukocytes to the endothelial lining is an integral step in the inflammatory response. Two types of selectins, CD62 and ELAM-1 (Endothelial-Leukocyte Adhesion Molecule 1) are bound by oligosaccharides containing α(1-3)fucosylated GlcNAc. Cell adhesion assays demonstrate that the addition of peptides containing such novel oligosaccharides (or the addition of the oligosaccharides alone) can bind the selectins and therefore reduce or prevent cellular adhesion. If cellular adhesion is reduced or prevented, the inflammatory response can also be reduced or prevented.

The discovery that 293 cells contain the enzymatic machinery necessary to produce such selectin-recognition ligands adds an important and useful tool to the medical community for the treatment of disease states linked to cell adhesion and the inflammatory response. Because of this discovery, glycoproteins and oligosaccharides produced in 293 cells may be used to treat inflammation or cell adhesion. Methods for using rHPC to treat a variety of health disorders are disclosed in Bang et al., U.S. Patent No. 4,775,624. The novel glycoproteins produced by 293 cells are not limited to recombinantly produced proteins in that some glycoproteins produced constitutively by 293 cells may also display the novel structures. Furthermore, the invention is not limited to the use of glycopeptides to prevent or reduce cell adhesion and inflammation. The skilled artisan will realize that the oligosaccharides can be removed from the glycoproteins by a wide variety of well-known procedures. The oligosaccharides can then be placed into a pharmaceutically acceptable diluant and administered to a patient undergoing cell adhesion or inflammation.

The skilled artisan will understand that the present invention is not limited to the production of the oligosaccharides of human protein C in 293 cells. Any peptide capable of being glycosylated can be transformed into 293 cells and expressed using techniques well known in the art. A list of such peptides includes, but is not limited to, human protein S, human thrombomodulin, derivatives of human protein C, tissue plasminogen activator and renin. The production of human protein S in 293 cells was disclosed in Yan, U.S. Patent No. 4,981,952. The genes encoding several derivatives of human protein C are disclosed in European Patent Application No. 91301450.2. The genes encoding natural and derivative forms of human thrombomodulin have been disclosed in European Patent Application No. 90308826.8 and

Wen et al.,1987, Biochemistry 26:4350. Any of these genes may be transformed into 293 cells which can then be cultured under conditions suitable for gene expression and peptide/oligosaccharide production.

The following examples are provided as a means of illustrating the present invention and are not to be construed as a limitation thereon.

Example 1

Production of Human Protein C in 293 Cells

Recombinant human protein C (rHPC) was produced in Human Kidney 293 cells by techniques well known to the skilled artisan such as those set forth in Yan, U.S. Patent No. 4,981,952. The gene encoding human protein C is disclosed and claimed in Bang et al., U.S. Patent No. 4,775,624. The plasmid used to express human protein C in 293 cells was plasmid pLPC which is disclosed in Bang et al., U.S. Patent No. 4,992,373. The construction of plasmid pLPC is also described in European Patent Publication No. 0 445 939 and in Grinnell et al., 1987, BioTechnology 5:1189-1192. Briefly, the plasmid was transfected into 293 cells, then stable transformants were identified and subcultured. The clones which demonstrated the highest level of expression were then grown in a 2 core Opticell fermenter in anchorage-dependent mode. Serum free media was used for all fermentations. To prepare the serum free media, the following ingredients were dissolved in 100 liters of high quality water:

| | |
|---|---|
| Dulbecco Modified Eagle media (#200-2010) | 1350 g |
| Ham's F12 media (#63N3085) | 478 g |
| Sodium Bicarbonate | 432 g |
| Dextrose | 468 g |
| Ethanolamine | 55 ml |
| Sodium Selenite 0.5M | 180 ml |
| Vitamin K (1%) | 180 ml |
| L-Glutamine | 81 g |

The solution was brought to 180 liters and the pH was adjusted to 7.2 with 3N HCl. After fermentation at 37 degrees C, the human protein C can be separated from the culture fluid by the techniques of Yan, U.S. Patent No. 4,981,952. The human protein C so produced can be used in the unactivated zymogen form or can be activated by procedures well known to one skilled in the art.

Example 2

Oligosaccharide Structure of rHPC Produced in 293 Cells

Many of the procedures used to elucidate the novel structure of the rHPC produced in 293 cells are described in Yan et. al., 1990, BioTechnology 8:655-651. The desalted and lyophilized rHPC was first reduced and carboxymethylated in 0.2M Tris buffer (pH8.6) containing 2mM EDTA and 6M guanidine HCl. This solution was thoroughly dialyzed against 50mM ammonium bicarbonate then lyophilized. The denatured rHPC was resuspended in sodium phosphate buffer (pH 8.6) then 12 units of N-glycanase (Genzyme) were added for every 8-9 mg of rHPC. The solution was incubated at 37 degrees C for 72 hours then the enzymatically released N-linked oligosaccharides were separated from the deglycosylated rHPC on a Bio-gel P6 column equilibrated with 50 mM ammonium bicarbonate. Fractions containing deglycosylated rHPC were monitored by $OD_{280nm}$.

The fractions containing the oligosaccharides, as monitored by glycosyl composition analysis, were pooled and lyophilized. The oligosaccharides were separated on the Dionex HPAE-PAD II system using a AS6 column (4.6 x 250mm) with an AG6 guard column which was equilibrated with 20 mM sodium acetate, 100 mM NaOH. After 3 minutes, the sodium acetate was increased to 60 mM in 20 minutes, further increased to 200mM in 120 minutes, increased to 400 mM in 40 minutes and again increased to 800mM in 5 minutes. The NaOH concentration was kept constant at 100mM and the column flow rate was 1 ml/min. Post-column 0.3 M NaOH was flowed at 0.5 ml/min.

An anion micromembrane suppressor was placed after the PAD detector. 30mM sulfuric acid flowing at 9 ml/min through the anion micromembrane suppressor was sufficient to neutralize and partially desalt the eluant from the AG6-AS6 column. Each oligosaccharide peak was further desalted with an OnGuard H cartridge (Dionex) prepared according to manufacturer's directions.

About 1-10 μg of desalted oligosaccharide was used as starting material to prepare PMAA derivatives for linkage analysis. The oligosaccharide was pre-reduced with 25 microliters of 10 mg/ml $NaBH_4$ in 1M $NH_4OH$ for 2 hours at room

temperature. The reaction was stopped with 20 microliters of glacial acetic acid, then the borate salts were removed by repeated coevaporation under nitrogen with 10% acetic acid in methanol. The reduced oligosaccharide was permethylated with a modified procedure of Ciucanu and Kerek, 1984, Carbohydr. Res. 131:209-217 as described by Costello and Vath, 1990, Meth. Enzymol. 193:738-755. The permethylated oligosaccharide was hydrolyzed in 2M TFA at 100 degrees C for 2 hours, then dried with rotor-evaporation. Deuteroreduction was achieved by adding $NaBD_4$ in 1M $NH_4OH$ at a concentration of 20 mg/ml at 40 degrees C for 1.5 hours. The reaction was stopped by neutralization with glacial acetic acid. The borate salts were removed by co-evaporation under nitrogen with 10% acetic acid in methanol. Acetylation was carried out by incubation with acetic anhydride at room temperature for 30 minutes. The final PMAA derivatives of the oligosaccharide were extracted with methylene chloride, concentrated by drying under nitrogen and redissolved in 10-20 microliters of ethyl acetate.

Gas Chromatography-Mass Spectrometry (GC-MS) was carried out using a Hewlett Packard GC5890-MSD. The PMAA derivatives were dissolved in ethyl acetate prior to injection on a DB-5 column (0.25mm x 30m, J & W Scientific) at a helium flow rate of 1 ml/min. The sample was injected at a column temperature of 80 degrees C which was kept for 2 minutes, then increased to 150 degrees at 30 degrees C/minute, then again increased from 150 degrees C to 240 degrees C at 2 degrees/minute, and kept at 240 degrees C for 10 minutes. The mass spectrometer was run at the EI quadruple mode. The GC-MS was calibrated with standards for elution times.

The structure of the oligosaccharide of Peak 1 was elucidated by glycosyl composition, linkage analysis and 1H-NMR. The structures of the other ten major oligosaccharides were predicted on the basis of glycosyl composition and linkage analysis. The oligosaccharide structures thus elucidated are set forth below.

The Carbohydrate Structure of Peak No. 1

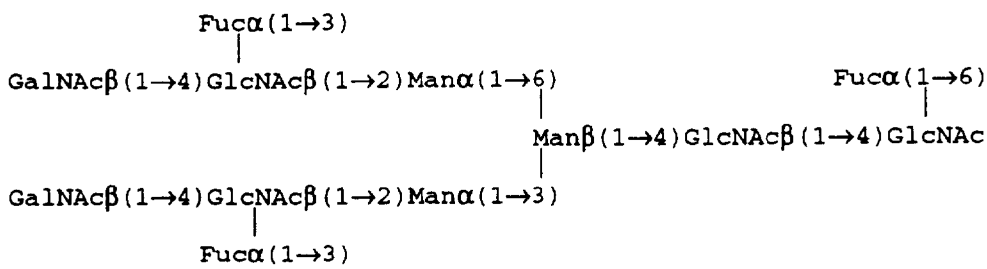

The Carbohydrate Structure of Peak No. 2

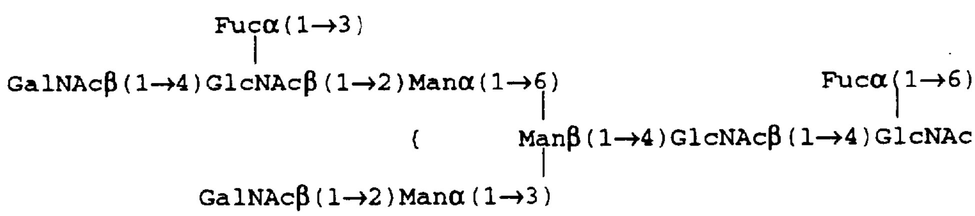

The Carbohydrate Structure of Peak No. 7

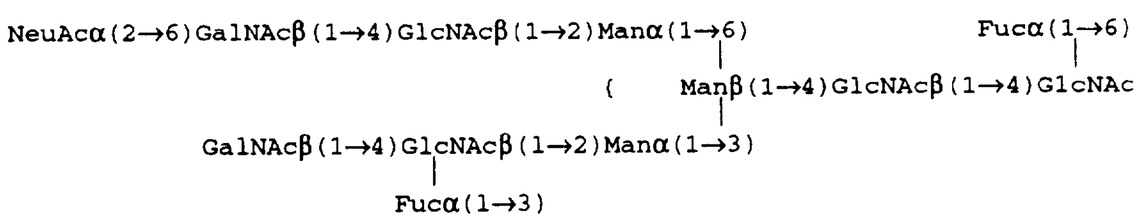

The Carbohydrate Structure of Peak 9

```
NeuAcα(2→6)GalNAcβ(1→4)GlcNAcβ(1→2)Manα(1→6)                    Fucα(1→6)
                                          |                           |
                                     {  Manβ(1→4)GlcNAcβ(1→4)GlcNAc
                                          |
              GalNAcβ(1→4)GlcNAcβ(1→2)Manα(1→3)
```

The Carbohydrate Structure of Peak 11

```
NeuAcα(2→3)Galβ(1→4)GlcNAcβ(1→2)Manα(1→6)                     Fucα(1→6)
                                     |                             |
                              {     Manβ(1→4)GlcNAcβ(1→4)GlcNAc
                                     |
           GalNAcβ(1→4)GlcNAcβ(1→2)Manα(1→3)
                         |
                   Fucα(1→3)
```

The Carbohydrate Structure of Peak 15

```
NeuAcα(2→6)GalNAcβ(1→4)GlcNAcβ(1→2)Manα(1→6)                  Fucα(1→6)
                                        |                          |
                                    {  Manβ(1→4)GlcNAc→(1→4)GlcNAc
                                        |
      NeuAcα(2→3)Galβ(1→4)GlcNAcβ(1→2)Manα(1→3)
```

The Carbohydrate Structure of Peak 19

```
NeuAcα(2→6)Galβ(1→4)GlcNAcβ(1→2)Manα(1→6)                  Fucα(1→6)
                                     |                          |
                                 {  Manβ(1→4)GlcNAcβ(1→4)GlcNAc
                                     |
  NeuAcα(2→3)Galβ(1→4)GlcNAcβ(1→2)Manα(1→3)
```

The Carbohydrate Structure of Peak 20

```
NeuAcα(2→6)GalNAcβ(1→4)GlcNAcβ(1→6)
                                   |
                          {      Manα(1→6)
                                   |       |
     NeuAcα(2→3)Galβ(1→4)GlcNAcβ(1→2)       |                  Fucα(1→6)
                                           |                       |
                          {              Manβ(1→4)GlcNAc→(1→4)GlcNAc
                                           |
     NeuAcα(2→3)Galβ(1→4)GlcNAcβ(1→2)Manα(1→3)
```

The Carbohydrate Structure of Peak 23

```
NeuAcα(2→6)GalNAcβ(1→4)GlcNAcβ(1→6)
                                  |
                              {  Manα(1→6)
                                  |      |
  NeuAcα(2→3)Galβ(1→4)GlcNAcβ(1→2)       {                  Fucα(1→6)
                                         |                     |
                            {          Manβ(1→4)GlcNAcβ(1→4)GlcNAc
                                         |
    NeuAcα(2→3)Galβ(1→4)GlcNAcβ(1→4)     {
                                  |      |
                              {  Manα(1→3)
                                  |
      NeuAcα(2→3)Galβ(1→4)GlcNAcβ(1→2)
```

The Carbohydrate Structure of Peak 24

```
NeuAcα(2→6)Galβ(1→4)GlcNAcβ(1→6)
                               |
                           {  Manα(1→6)
                               |      |
NeuAcα(2→3)Galβ(1→4)GlcNAcβ(1→2)      {                  Fucα(1→6)
                                      |                     |
                           {        Manβ(1→4)GlcNAcβ(1→4)GlcNAc
                                      |
    NeuAcα(2→3)Galβ(1→4)GlcNAcβ(1→4)  {
                                  |   |
                              { Manα(1→3)
                                  |
    NeuAcα(2→3)Galβ(1→4)GlcNAcβ(1→2)
```

The Carbohydrate Structure of Peak 25

```
NeuAcα(2→3)Galβ(1→4)GlcNAcβ(1→6)
                               |
                          {  Manα(1→6)
                               |      |
  NeuAcα(2→3)Galβ(1→4)GlcNAcβ(1→2)    {                   Fucα(1→6)
                                      |                      |
                            {       Manβ(1→4)GlcNAcβ(1→4)GlcNAc
                                      |
NeuAcα(2→3)Galβ(1→4)GlcNAcβ(1→4)      {
                               |      |
                          {  Manα(1→3)
                               |
NeuAcα(2→3)Galβ(1→4)GlcNAcβ(1→2)
```

Example 3

In vitro Cell Adhesion Assays

Human Umbilical Vein Endothelium Cells (HUVEC) or Human Aortic Endothelium (HAE) were obtained from Clonetics (San Diego) and grown in the EBM medium supplied by Clonetics. Cells were plated in 96 well plates at a density to obtain confluent monolayers following overnight incubation at 37 degrees C. Monolayers were incubated with or without 20 nanograms Tumor Necrosis Factor (TNF) for 4 to 6 hours prior to the binding assay in a total volume of 100 to 150 microliters. To test for inhibition of binding, samples of protein C or its isolated carbohydrates were added to triplicate wells in volumes up to 20 microliters in Phosphate Buffered Saline (PBS) or water, and then incubated for an additional 20 to 25 minutes. Following incubations, tritium-labelled U937 cells were added in 50 microliter volumes at from 1 to 3 x 10(6) cells per well. The U937 cells were tritium labelled by the addition of 3H-thymidine to a final concentration of 1 microcurie per milliliter, followed by 18 to 20 hours incubation. Cells were washed with PBS prior to use to remove excess label. After a 20 minute incubation of the labeled U937 cells with the endothelial cells, the wells were aspirated and washed four times with calcium-containing PBS. The monolayer and adherent U937 cells were solubilized by the addition of 0.25% SDS/0.1 N NaOH for 5 minutes with agitation. The level of binding was determined by scintillation counting of the solubilized cells.

Table I

| Experiment# | aPC µg/ml | % inhibition[a] |
|---|---|---|
| 1(HUVECS)[b] | 32 | None |
| 1(HUVECS) | 120 | 70(12)[c] |
| 2(HAE) | 120 | None |
| 2(HAE) | 240 | 77(30) |
| 3(HAE) | 120 | 54(18) |

[a]100% inhibition is the difference between U937 binding in the presence and absence of pretreatment of the endothelial cells with TNF

[b]cell line

[c]numbers in parenthesis indicate standard error

Table II

| Experiment# | oligosaccharide (μM) | % inhibition[a] |
|---|---|---|
| 1(HAE)[b] | 5.75 | None |
| 1(HAE) | 11.5 | 22(0.5)[c] |
| 1(HAE) | 23 | 16(4) |
| 2(HUVECS) | 17.25 | 19[d] |
| 2(HUVECS) | 34.5 | 63 |

[a]100% inhibition is the difference between U937 binding in the presence and absence of pretreatment of the endothelial cells with TNF

[b]cell line

[c]numbers in parenthesis indicate standard error

[d]no error calculated from single point determinations

## Claims

1. An oligosaccharide of the structure

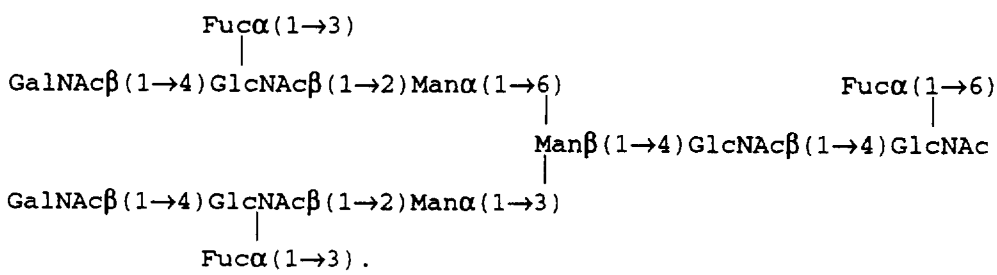

2. An oligosaccharide of the structure

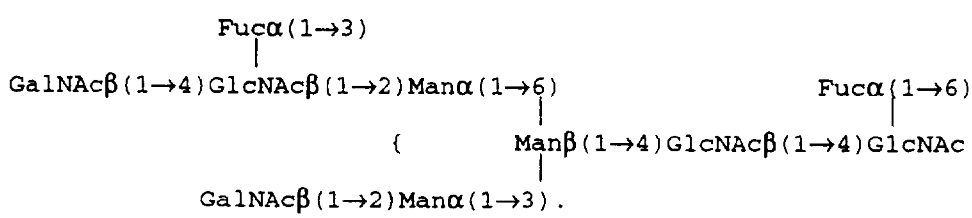

3. An oligosaccharide of the structure

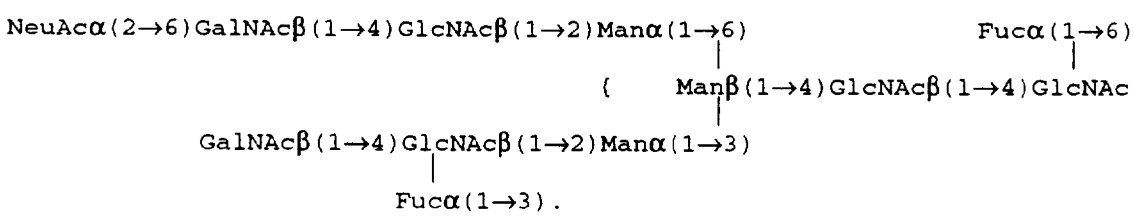

**4.** An oligosaccharide of the structure

```
NeuAcα(2→3)Galβ(1→4)GlcNAcβ(1→2)Manα(1→6)                 Fucα(1→6)
                                          |                   |
                                  (      Manβ(1→4)GlcNAcβ(1→4)GlcNAc
                                          |
         GalNAcβ(1→4)GlcNAcβ(1→2)Manα(1→3)
                        |
                     Fucα(1→3).
```

**5.** A pharmaceutical formulation comprising as an active ingredient an oligosaccharide as claimed in any one of Claims 1 to 4, associated with one or more pharmaceutically acceptable carriers, excipients or diluents therefor.

**6.** An oligosaccharide as claimed in any of Claims 1 to 4 for use as an antiinflammatory agent.

**7.** An oligosaccharide as claimed in any of Claims 1 to 4 for use to prevent inflammation.

**8.** An oligosaccharide as claimed in any of Claims 1 to 4 for use to reduce cellular adhesion.

**9.** An oligosaccharide as claimed in any of Claims 1 to 4 for use to prevent cellular adhesion.

**Patentansprüche**

**1.** Oligosaccharid der Struktur

```
                Fucα(1→3)
                  |
GalNAcβ(1→4)GlcNAcβ(1→2)Manα(1→6)                 Fucα(1→6)
                                  |                   |
                                 Manβ(1→4)GlcNAcβ(1→4)GlcNAc
                                  |
GalNAcβ(1→4)GlcNAcβ(1→2)Manα(1→3)
                  |
               Fucα(1→3).
```

**2.** Oligosaccharid der Struktur

```
                Fucα(1→3)
                  |
GalNAcβ(1→4)GlcNAcβ(1→2)Manα(1→6)                 Fucα(1→6)
                                  |                   |
                        (        Manβ(1→4)GlcNAcβ(1→4)GlcNAc
                                  |
               GalNAcβ(1→2)Manα(1→3).
```

**3.** Oligosaccharid der Struktur

```
NeuAcα(2→6)GalNAcβ(1→4)GlcNAcβ(1→2)Manα(1→6)                 Fucα(1→6)
                                             |                   |
                                     (      Manβ(1→4)GlcNAcβ(1→4)GlcNAc
                                             |
           GalNAcβ(1→4)GlcNAcβ(1→2)Manα(1→3)
                          |
                       Fucα(1→3).
```

**4.** Oligosaccharid der Struktur

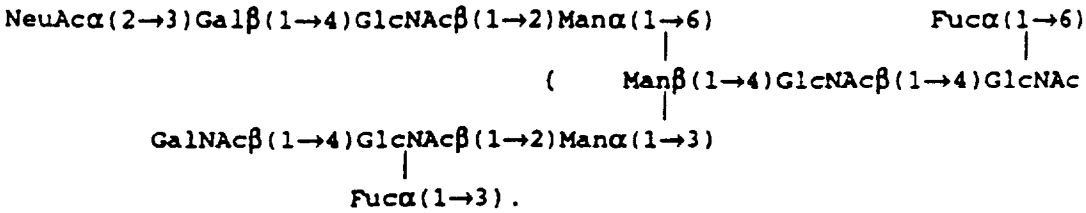

5. Pharmazeutische Formulierung, die als Wirkstoff ein Oligosaccharid nach einem der Ansprüche 1 bis 4 zusammen mit einem oder mehreren pharmazeutisch annehmbaren Trägern, Hilfsstoffen oder Verdünnungsmitteln hierfür enthält.

6. Oligosaccharid nach einem der Ansprüche 1 bis 4 zur Verwendung als Mittel gegen Entzündungen.

7. Oligosaccharid nach einem der Ansprüche 1 bis 4 zur Verwendung bei der Verhinderung einer Entzündung.

8. Oligosaccharid nach einem der Ansprüche 1 bis 4 zur Verwendung bei der Reduzierung der zellulären Adhäsion.

9. Oligosaccharid nach einem der Ansprüche 1 bis 4 zur Verwendung bei der Verhinderung der zellulären Adhäsion.

**Revendications**

1. Oligosaccharide de structure

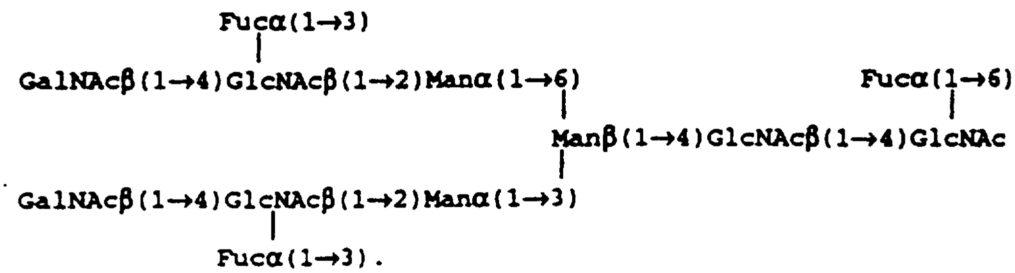

2. Oligosaccharide de structure

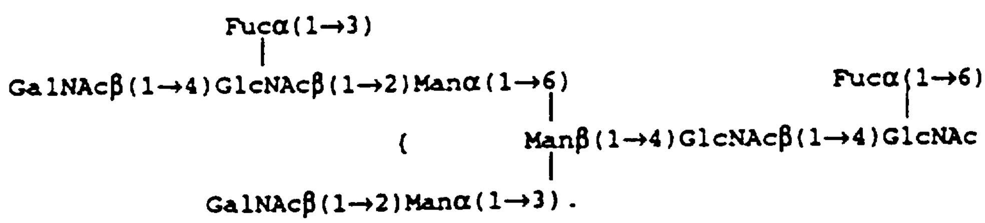

3. Oligosaccharide de structure

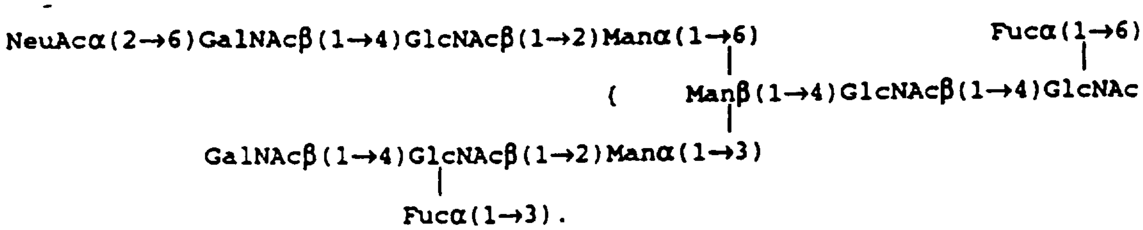

4. Oligosaccharide de structure

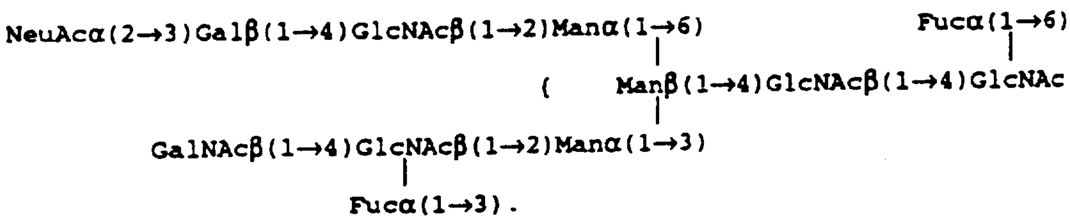

5. Formulation pharmaceutique comprenant comme ingrédient actif, un oligosaccharide tel que revendiqué dans l'une quelconque des revendications 1 à 4, en association avec un ou plusieurs supports, excipients ou diluants pharmaceutiquement acceptables pour l'ingrédient actif.

6. Oligosaccharide selon l'une quelconque des revendications 1 à 4, à utiliser comme agent anti-inflammatoire.

7. Oligosaccharide selon l'une quelconque des revendications 1 à 4, à utiliser pour empêcher l'inflammation.

8. Oligosaccharide selon l'une quelconque des revendications 1 à 4, à utiliser pour réduire l'adhésion cellulaire.

9. Oligosaccharide selon l'une quelconque des revendications 1 à 4, à utiliser pour empêcher l'adhésion cellulaire.

FIG.1

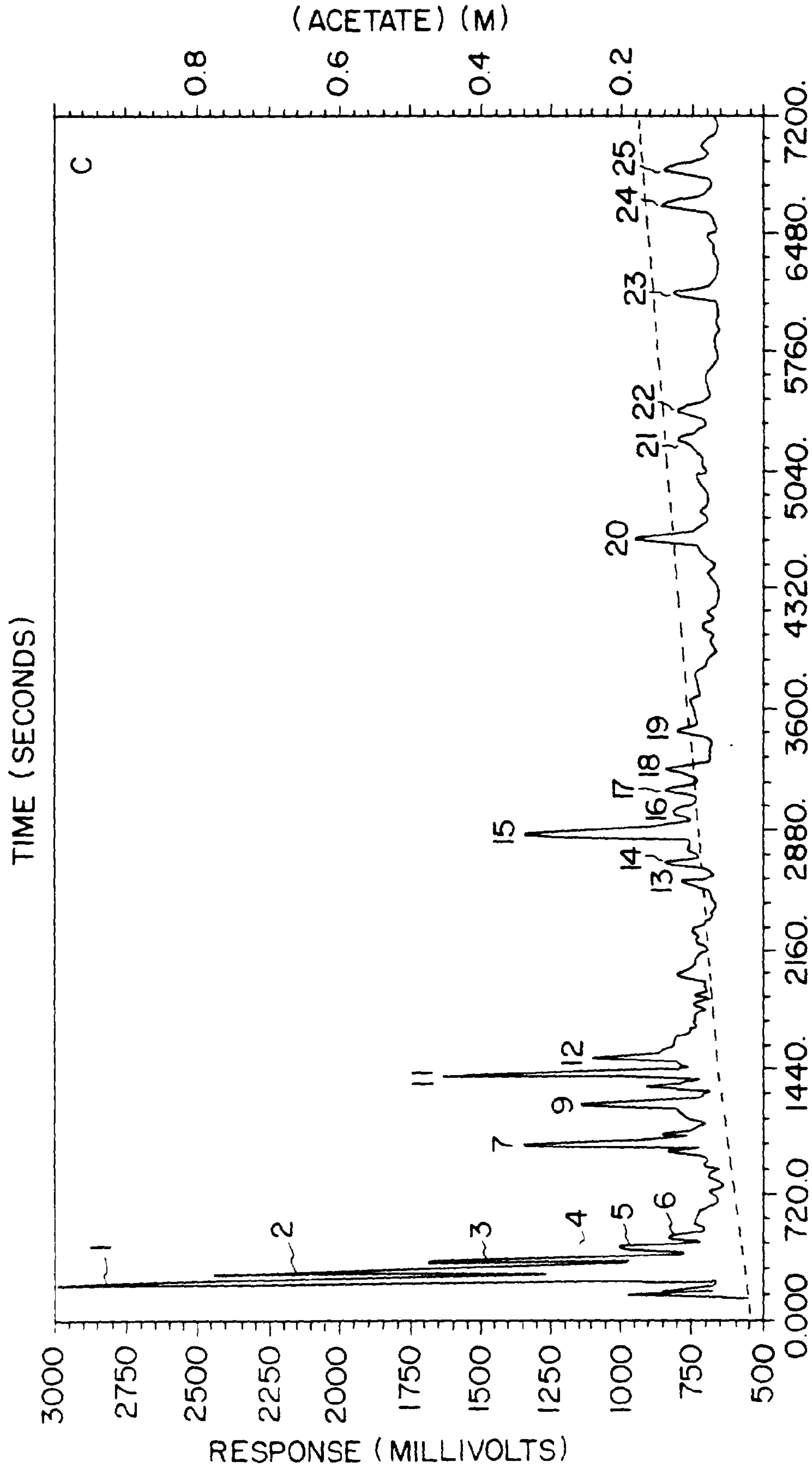
TIME (SECONDS)
(ACETATE) (M)
0.8
0.6
0.4
0.2
C
RESPONSE (MILLIVOLTS)
3000
2750
2500
2250
2000
1750
1500
1250
1000
750
500
0.000
720.0
1440.
2160.
2880.
3600.
4320.
5040.
5760.
6480.
7200.
1
2
3
4
5
6
7
9
11
12
13
14
15
16
17
18
19
20
21
22
23
24
25